# FASCICULE DE BREVET EUROPEEN

(11) **EP 3 609 556 B1**
(45) Date de publication et mention de la délivrance du brevet: **07.07.2021**
(21) Numéro de dépôt: 18722121.3
(22) Date de dépôt: 06.04.2018
(51) Int. Cl.: A61M 5/145, A61M 5/155

(54) **DISPOSITIF D'INJECTION AUTOMATIQUE DE PRODUIT FLUIDE**
AUTOMATISCHE INJEKTIONSVORRICHTUNG FÜR FLUIDPRODUKTE
AUTOMATIC FLUID PRODUCT INJECTION DEVICE

(30) Priorité: 10.04.2017 FR 1753093
(43) Date de publication de la demande: 19.02.2020
(73) Titulaire: Aptar France SAS, 27110 Le Neubourg (FR)
(72) Inventeur: REEVES, Sam, Cambride Cambridgeshire CB4 0WS (GB)
(74) Mandataire: CAPRI
(86) Numéro de dépôt international: PCT/FR2018/050859
(87) Numéro de publication internationale: WO 2018/189459

(56) Documents cités:
- WO-A2-2008/057223
- US-A- 4 070 859
- US-A- 5 222 362

## Description

La présente invention concerne un dispositif d'injection automatique de produit fluide.

Les dispositifs d'injection automatique de produit fluide sont bien connus. Ils comportent notamment les autoinjecteurs, dans lesquels le contenu d'un réservoir, généralement une seringue, est automatiquement injecté au moyen d'un système d'actionnement comprenant généralement un ressort chargé et qui lors du déclenchement va déplacer un piston dans le réservoir pour injecter le produit fluide.

Ces dispositifs de l'art antérieur peuvent présenter des problèmes, notamment lorsque les volumes à distribuer sont importants, lorsque le produit fluide est relativement visqueux ou lorsque plusieurs produits fluides doivent être associés dans un même traitement.

Les documents US5222362, WO2008057223 et US4070859 décrivent des dispositifs de l'état de la technique.

La présente invention a pour but de fournir un dispositif d'injection automatique qui ne reproduit pas les inconvénients susmentionnés.

La présente invention a aussi pour but de fournir un dispositif d'injection automatique qui permet la distribution automatique de produit fluide, même à des volumes et/ou viscosités importants.

La présente invention a également pour but de fournir un dispositif d'injection automatique de produit fluide qui est simple et peu coûteux à fabriquer et à assembler.

La présente a donc pour objet un dispositif d'injection automatique de produit fluide comportant un corps fixé à un support destiné à venir au contact d'une zone à injecter, ledit corps contenant un ou plusieurs réservoirs de produit fluide contenant chacun un piston d'injection, un ensemble d'aiguille comportant une aiguille d'injection destinée à pénétrer dans la zone à injecter, et un mécanisme d'injection, ledit mécanisme d'injection comportant une capsule d'actionneur contenant un matériau expansible et des moyens de chauffage pour chauffer et ainsi provoquer l'expansion dudit matériau expansible pour déplacer ledit piston dans ledit réservoir et ainsi injecter le produit fluide dans ladite zone à injecter à travers ladite aiguille d'injection, ledit matériau expansible comprenant des microsphères expansibles thermiquement.

Avantageusement, lesdites microsphères expansibles thermiquement comprennent des bulles thermoplastiques microscopiques avec un hydrocarbure encapsulé.

Avantageusement, lesdites bulles thermoplastiques microscopiques, après chauffage à 60-90°C, se dilatent et subissent un changement de phase en gaz, permettant l'expansion desdites microsphères expansibles thermiquement à 5-70 fois leur volume d'origine.

Avantageusement, ledit dispositif comporte un ensemble d'amorçage comportant un actionneur d'amorçage et une ou plusieurs aiguille(s) d'amorçage destinée(s) à pénétrer dans le ou les réservoirs.

Avantageusement, ledit support comporte un autocollant pour se fixer sur la zone à injecter.

Avantageusement, chaque réservoir contient entre 1 et 10 ml de produit fluide, avantageusement environ 3 ml.

Avantageusement, ledit corps comporte plusieurs réservoirs, notamment trois.

Avantageusement, les produits fluides contenus dans lesdits réservoirs sont distribués simultanément, en étant mélangé en amont de ladite aiguille d'injection.

Avantageusement, les produits fluides contenus dans lesdits réservoirs sont distribués successivement.

Avantageusement, ledit dispositif comporte des moyens d'alimentation de puissance, avantageusement une pile et/ou un accumulateur d'énergie électrique.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en perspective éclatée d'un dispositif d'injection automatique selon un mode de réalisation avantageux,
- les figures 2 et 3 sont des vues schématiques de côté du dispositif de la figure 1, respectivement avant et après amorçage,

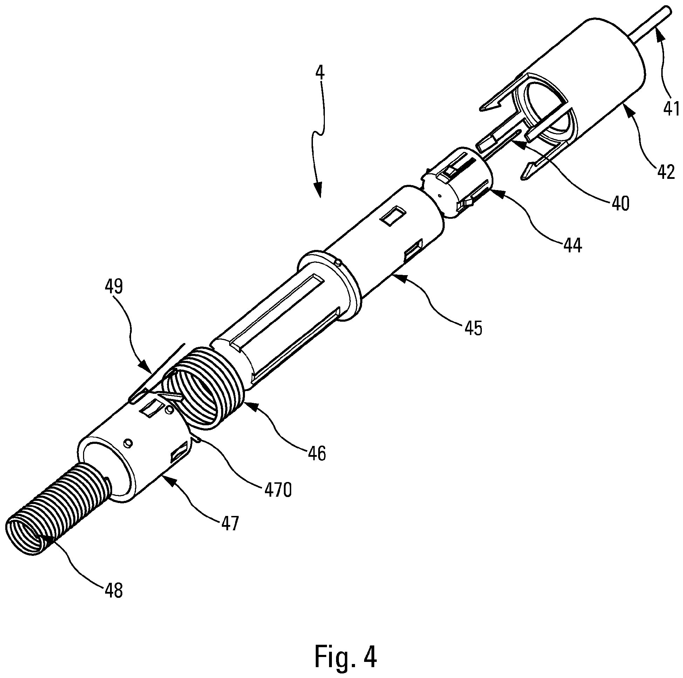

- la figure 4 est une vue schématique en perspective éclatée d'un ensemble d'aiguille selon un mode de réalisation avantageux,
- les figures 5 à 8 sont des vues schématiques de côté de la séquence d'actionnement de l'ensemble d'aiguille de la figure 4,

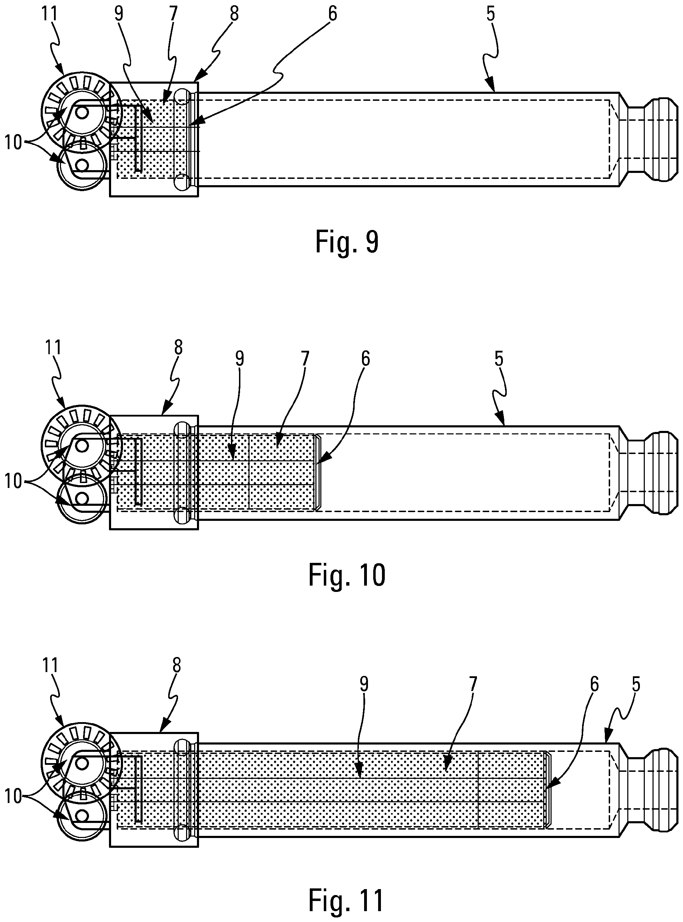

- les figures 9 à 11 sont des vues schématiques de côté de la séquence d'actionnement du dispositif de la figure 1,
- la figure 12 est un schéma explicatif du fonctionnement des microsphères d'hydrocarbure encapsulées, et
- la figure 13 est un schéma illustrant le volume d'expansion des microsphères d'hydrocarbure encapsulées en fonction de la température.

L'invention concerne un dispositif d'injection automatique particulièrement adapté à distribuer des volumes relativement importants de produit fluide, typiquement de l'ordre de quelques millilitres, typiquement de 1 à 10 ml, par exemple 3 ml. Le dispositif de l'invention est aussi adapté à distribuer des produits fluides relativement visqueux.

Le dispositif est de préférence jetable et peut fonctionner avec les étapes d'utilisation suivantes:
1) l'utilisateur retire l'emballage et fixe le dispositif sur une zone à injecter, par exemple au moyen d'un autocollant prévu à cet effet;
2) l'utilisateur appuie sur un bouton d'actionnement 200 pour actionner le dispositif, ce qui provoque notamment l'amorçage du dispositif, l'insertion de l'aiguille d'injection dans la zone à injecter, l'administration du produit fluide puis la rétractation de l'aiguille d'injection;
3) l'utilisateur est alerté de l'achèvement du processus, il détache l'appareil du corps et le jette.

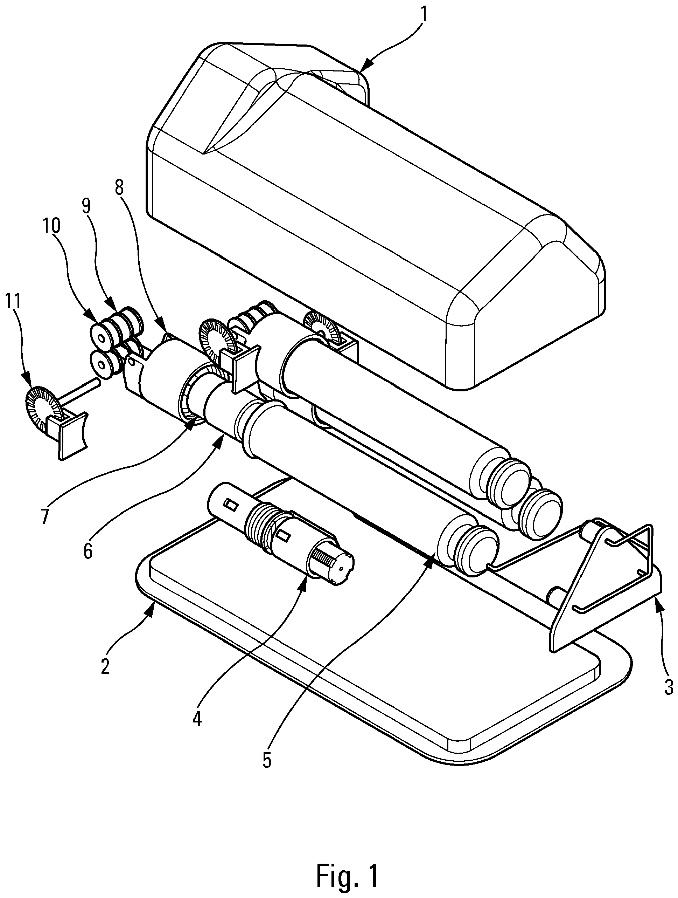

Sur la figure 1, il est représenté un dispositif d'injection automatique selon un mode de réalisation avantageux.

Le dispositif comprend un corps 1 fixé à un support 2 destiné à venir au contact de la zone à injecter, ledit corps contenant un ou plusieurs réservoirs 5 de produit fluide contenant chacun un piston d'injection 6, un ensemble d'amorçage 3 comportant un actionneur d'amorçage et une ou plusieurs aiguille(s) d'amorçage 31 destinée(s) à pénétrer dans le ou les réservoirs 5, un ensemble d'aiguille 4 comportant une aiguille d'injection 40 (non représentée sur la figure 1) destinée à pénétrer dans la zone à injecter, un mécanisme d'injection 7-11, et des moyens d'alimentation de puissance (non représentés).

L'alimentation peut par exemple être une pile ou un accumulateur d'énergie électrique.

Le mécanisme d'injection dans cet exemple est du type thermique.

La forme de réalisation du système montre un ensemble de trois réservoirs 5 avec un ensemble d'amorçage 3 et un ensemble d'aiguille 4.

L'actionnement du dispositif provoque une série de processus internes contrôlés comme suit:
1) Actionnement de l'ensemble d'amorçage 3, qui peut également inclure un mécanisme d'indication (micro-interrupteur, etc.) pour alerter le contrôleur d'amorçage que le perçage de la membrane du ou des réservoirs est terminé.
2) Insertion de l'aiguille d'injection via un mécanisme à commande électronique ; ce système peut également comporter une indication pour indiquer l'achèvement du piquage au contrôleur d'injection.
3) Déclenchement du mécanisme d'injection.
4) Rétraction de l'aiguille d'injection via le mécanisme à commande électronique, avec un mécanisme d'indication (par exemple micro-interrupteur, temporisateur à boucle ouverte) pour indiquer à l'utilisateur (visuellement, par exemple via une diode, et/ou de manière audible, par exemple via un signal sonore) que le dispositif peut être retiré du corps.

La séquence décrite ci-dessus peut impliquer la présence d'un petit volume d'air dans les ensembles d'amorçage et d'aiguille, et notamment dans les aiguilles d'amorçage et d'injection, auquel cas le produit fluide devrait être injecté dans le tissu sous-cutané lors de l'utilisation du dispositif.

Pour les applications où un tel volume d'air, même très petit, ne serait pas acceptable, la séquence d'actionnement du dispositif peut comporter l'étape supplémentaire 1.1:
1.1) Après amorçage et avant insertion de l'aiguille d'injection dans la zone à injecter, actionnement préalable du mécanisme d'injection pour distribuer un petit volume contrôlé du produit fluide contenu dans le réservoir pour expulser l'air contenu dans les ensembles d'amorçage et d'aiguille.

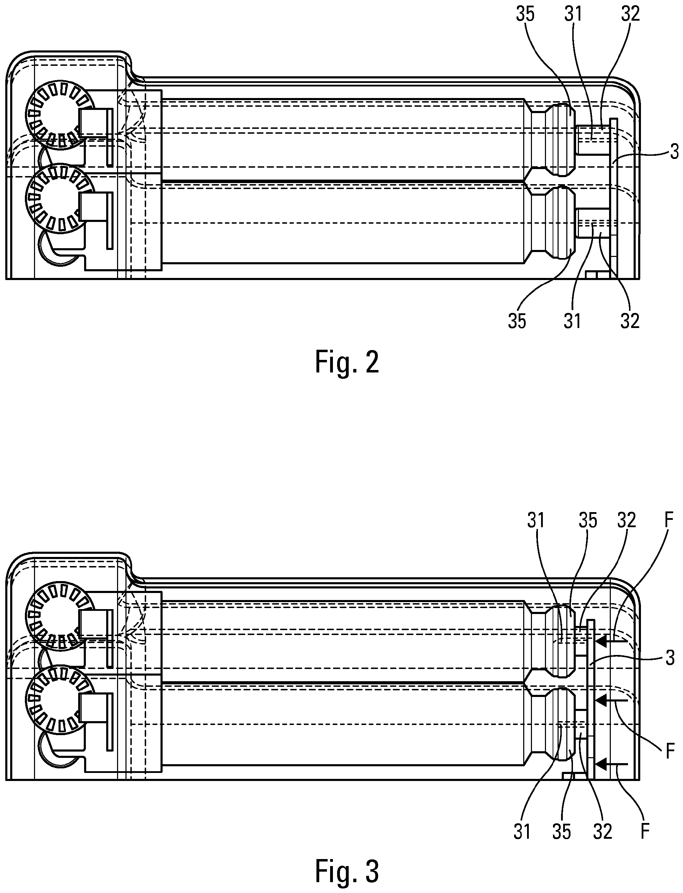

Avantageusement, chaque réservoir 5 est obturé avant actionnement par une membrane 35 formant septum, destinée à être percée par une aiguille d'amorçage 31 respective lors de l'actionnement, comme visible sur les figures 2 et 3.

L'ensemble d'amorçage se déplace de préférence sur un seul axe de mouvement commandé par un actionneur d'amorçage linéaire (non représenté).

Les actionneurs d'amorçage envisagés peuvent être d'un type quelconque approprié, notamment un actionneur en alliage à mémoire de forme (AMF), un actionneur électromagnétique à solénoïde ou un actionneur électromagnétique à vis sans fin.

L'actionnement du dispositif entraîne les actions suivantes de l'ensemble d'amorçage:
1) Au repos, avant actionnement, chaque aiguille d'amorçage 31 est obturée par un masque 32 respectif, par exemple en élastomère, pour maintenir la stérilité de l'aiguille d'amorçage 31.
2) L'activation de l'actionneur d'amorçage fait avancer ledit ensemble d'amorçage 3 contre la membrane 35 de chaque réservoir 5, comme représenté par les flèches F sur la figure 3.
3) Chaque masque d'aiguille 32 est comprimé contre le réservoir 5 respectif, et se déforme pour permettre une progression continue de l'aiguille d'amorçage 3 respective à travers la membrane 35 respective pour pénétrer dans le réservoir 5 respectif.
4) L'actionneur d'amorçage maintient la position de l'aiguille d'amorçage 31 pendant la durée de la distribution du médicament d'une quelconque manière appropriée.

Le mode de réalisation décrit ci-dessus offre notamment les avantages suivants:
1) Au repos, chaque aiguille d'amorçage 31 reste dans un environnement stérile protégé.
2) L'ensemble d'amorçage 3 fonctionne avec un ou plusieurs réservoirs 5 ; ainsi, si une ou plusieurs aiguilles d'amorçage 31 restent inutilisées ou ne pénètrent pas dans un réservoir 5 respectif, le système reste fermé et fonctionnel pour la ou les aiguilles d'amorçage restantes.

Après que le dispositif a été amorcé, l'aiguille d'injection 40 de l'ensemble d'aiguille 4 est insérée dans la zone à injecter du patient au moyen d'un actionneur d'insertion.

Si plusieurs réservoirs 5 sont utilisés, comme représenté dans les exemples des figures 1 à 3, les aiguilles d'amorçage 31 de tous les réservoirs 5 sont couplées à une seule aiguille d'injection 40.

L'ensemble d'aiguille 4 comporte avantageusement, outre l'aiguille d'injection 40, un manchon d'aiguille 41, qui est optionnel, une bague de rétractation 42, un support d'aiguille 44, une bague d'insertion 45, un ressort de rétractation 46, une bague d'actionnement 47, un ressort d'insertion 48 et un actionneur d'insertion en alliage à mémoire de forme (AMF), ici réalisé sous la forme d'un fil 49.

Les matériaux AMF, comme l'alliage nickel-titane (Nitinol), subissent un changement de phase martensitique lorsqu'ils sont chauffés. Ce chauffage peut être avantageusement appliqué par effet Joule. Ce changement de phase provoque une modification des dimensions physiques du matériau, à savoir dans le mode de réalisation décrit, une contraction de la longueur du fil 49.

Les contraintes maximales typiques qui peuvent être générées dans les fils AMF sont d'environ 10%, et le mode de réalisation décrit utilise avantageusement une boucle pour générer le déplacement requis dans les composants décrits.

Le fil AMF 49 revient à son état physique précédent lors du refroidissement, ici obtenu par convection naturelle après suppression du courant d'activation. Des éléments à ressort pour ramener le fil à sa forme initiale sont représentés ici par une série de languettes courbes 470 intégrées dans la bague d'actionnement 47.

Tant le ressort d'insertion d'aiguille 48 que le ressort de rétraction d'aiguille 46 sont à l'état comprimé et retenus par des moyens de verrouillage respectifs.

L'activation de l'actionneur AMF 49 par le chauffage par effet Joule pour atteindre 50% de la contraction disponible provoque le déplacement de la bague d'actionnement 47 vers l'épaulement 451 de la bague d'insertion 45 à laquelle une extrémité de l'actionneur AMF 49 est fixée.

Dans l'exemple représenté, ce déplacement entraîne la déformation de pattes d'encliquetage 440 du support d'aiguille 44, libérant le support d'aiguille 44 et permettant l'extension du ressort d'insertion 48.

L'extension du ressort d'insertion 48 amène le support d'aiguille 44 et l'aiguille d'injection 40 à traverser l'alésage central de la bague d'insertion 45.

Cette extension du ressort d'insertion 48 est utilisée pour faire pénétrer l'aiguille d'injection 40 dans le patient, où elle restera après l'extension du fil de l'actionneur AMF 49.

Dans l'exemple représenté, le déplacement du support d'aiguille 44 est arrêté par l'engagement des pattes d'encliquetage 440 avec des découpes 450 dans le corps de la bague d'insertion 45.

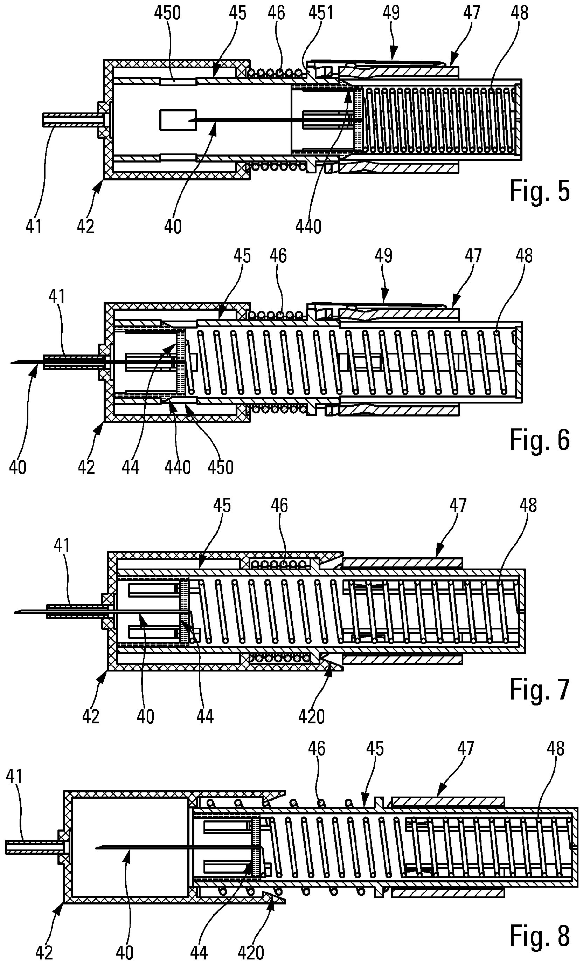

Le mécanisme d'insertion d'aiguille, à l'état inséré de l'aiguille, est représenté sur la figure 7.

Ici, la vue en coupe est tournée de 45° par rapport à la figure 6 de façon à montrer la fonction des pattes d'encliquetage 420 de la bague de rétractation 42.

L'activation de l'actionneur AMF 49 (non représenté ici) par le chauffage par effet Joule pour atteindre 100% de sa contraction disponible provoque le déplacement de la bague d'actionnement 47 vers les pattes d'encliquetage 420 de la bague de rétractation 42.

Dans l'exemple représenté, ce déplacement entraîne la déformation des pattes d'encliquetage 420 de la bague de rétractation 42, pour libérer la bague d'insertion 45.

La bague d'insertion 45 et l'ensemble interconnecté formé du support d'aiguille 44 et de l'aiguille d'injection 40 sont entraînés par l'extension du ressort de rétractation 46, actionné par la libération des pattes d'encliquetage 420.

Cette action rétracte l'aiguille 40 hors du corps de l'utilisateur et le mécanisme restera dans cette position après désactivation du fil d'actionneur 49.

Après insertion de l'aiguille d'injection, le mécanisme d'injection est actionné.

Dans l'exemple représenté, le mécanisme d'injection comporte une capsule d'actionneur 7 contenant un matériau expansible, un corps d'actionneur 8, et des moyens de chauffage 9, notamment un fil à effet Joule enroulé sur une bobine respective 10.

Dans l'exemple représenté, le mécanisme d'injection comprend en outre un dispositif 11 (par exemple un encodeur optique) pour mesurer le déplacement angulaire d'au moins une bobine 10.

Le moteur principal pour l'administration du produit fluide est la pression créée dans le réservoir 5 par la dilatation volumétrique du matériau contenu dans la capsule d'actionneur 7.

Le mode de fonctionnement peut être décrit comme suit:
1) Au repos, la capsule d'actionneur 7 est retenue dans le corps d'actionneur 8 fixé sur le rebord du réservoir 5. Le corps 8 supporte deux bobines 10 de fils de chauffage 9 et l'encodeur 11. Les fils de chauffage 9 ont une partie enroulée autour de la bobine et s'étendent également dans le corps 8 de l'actionneur, à travers la capsule d'actionneur 7, pour se fixer sur le piston 6 d'un réservoir 5 respectif.
2) L'application d'un courant électrique aux fils de chauffage 9 entraîne leur chauffage. Les fils 9 sont placés avec un écartement suffisant pour établir une température uniforme à l'intérieur de l'actionneur, typiquement de 60°C à 90°C. En atteignant cette température, le contenu de la capsule d'actionneur 7 subit une expansion volumétrique importante.
3) La dilatation volumétrique de la capsule d'actionnement 7 provoque le déplacement du piston 6 sur la longueur du réservoir 5. Le déplacement du piston 6 exerce une tension sur les fils chauffants 9 qui peuvent être déployés depuis les bobines 10 car celles-ci sont libres de tourner. Ce mécanisme permet en outre une expansion continue de la capsule d'actionneur 7, car le chauffage est maintenu dans tout le volume du matériau expansible.
4) La température à l'intérieur de l'actionneur et l'expansion associée sont modulées par la commande du courant électrique appliqué. Ceci est déterminé par le contrôle du déplacement du piston mesuré par l'intermédiaire du dispositif encodeur 11. Une fois que suffisamment de produit fluide a été distribué du réservoir 5, le processus est terminé par suppression du courant appliqué.

Un moyen pour fournir une expansion volumétrique de la capsule d'actionneur 7 au moyen d'un stimulus thermique est l'utilisation de microsphères expansibles thermiquement. Il s'agit de bulles thermoplastiques microscopiques (diamètre d'environ 12µm) avec un hydrocarbure encapsulé.

Le liquide encapsulé est un hydrocarbure saturé qui, après chauffage à 60-90°C, se dilate et subit un changement de phase en gaz. Le milieu d'encapsulation est un thermoplastique qui se ramollit sous l'action de chauffage permettant l'expansion de la sphère à 5-70 fois son volume d'origine (cf. le document Nishiyama, Y., Nobuyuki, U. & Sato, C., Comportement de démantèlement et résistance d'adhésif démontable incluant des particules thermiquement expansibles, Int. J. Adhésion & Adhesives, V.23, Iss. 5, pages 377-382, (2003)). Ici, l'application d'une pression externe de 1 MPa devrait entraîner une expansion volumétrique totale d'un facteur 9, fournissant un déplacement du piston de 45 mm à partir d'une longueur initiale de capsule d'actionneur 7 de 5 mm de long.

A la fin de la course de l'actionneur, la chaleur est supprimée. Ceci fait augmenter le module du matériau thermoplastique encapsulant, empêchant la contraction des microsphères de leur état étendu. Cela signifie que la course de l'actionneur ne peut pas être réutilisée.

Des microsphères d'hydrocarbures encapsulées sont disponibles en tant que matériau de remplissage par exemple auprès des sociétés AkzoNobel (Suède) et Matsumoto Yushi-Seiyaku Co. Ltd. (Japon).

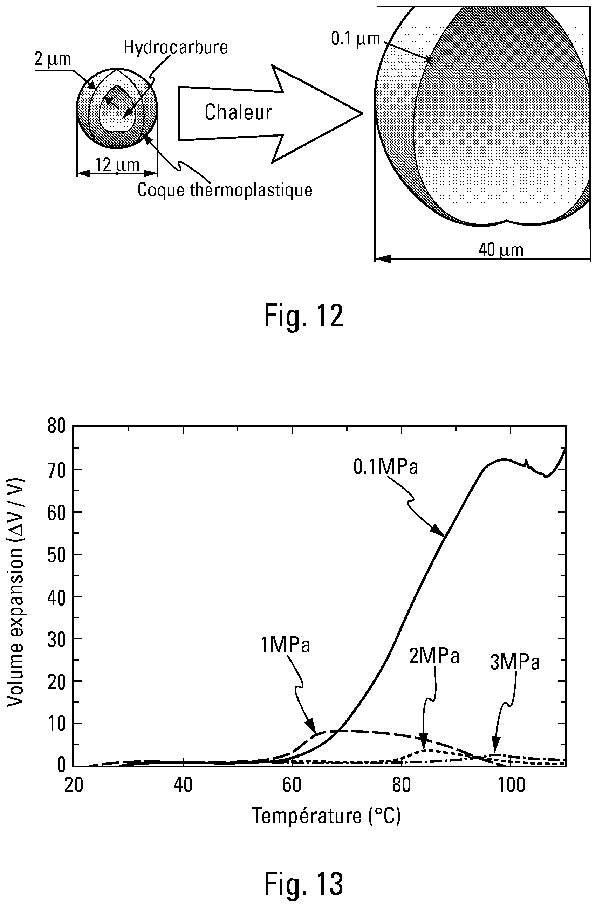

Le tableau de la figure 13 illustre la mesure du rapport de dilatation volumétrique des microsphères encapsulées à diverses pressions externes (source: Nishiyama et al., 2003).

Le schéma de la figure 12 montre une description des microsphères disponibles chez AkzoNobel Gamme Expanncell (source: AkzoNobel, courte introduction aux microsphères d'Expancell).

Lorsque la distribution du produit fluide est terminée, l'actionneur d'insertion 8 rétracte l'aiguille dans le dispositif. La fin de la distribution du produit fluide peut être identifiée par un contrôle mécanique et/ou logiciel, par exemple après que le mécanisme d'entrainement 7 est complètement étendu.

Le dispositif représenté sur la figure 1 comporte trois réservoirs 3. Les trois mécanismes d'entrainement peuvent être actionnés simultanément, pour distribuer simultanément les contenus des trois réservoirs, qui sont alors mélangés en amont de l'aiguille d'injection 40. En variante, les trois mécanismes d'entrainement peuvent être actionnés successivement, pour distribuer successivement les contenus des trois réservoirs. Ces actionnements successifs peuvent être déclenchés séparément, mais on pourrait aussi prévoir un seul actionnement qui déclenche automatiquement la séquence de distribution. Un mélange de ces deux variantes est aussi possible, par exemple une distribution en deux temps, avec d'une part la distribution du contenu d'un réservoir et d'autre part la distribution simultanée du mélange des deux autres réservoirs.

L'utilisation d'un dispositif à réservoirs multiples permet de fournir notamment les avantages suivants:
- dispositif unique pour plusieurs types de produits fluides, qui peut nécessiter la distribution de volumes différents;
- possibilité de distribuer des cocktails ou mélange de plusieurs produits fluides;
- possibilités d'associer des agents réducteurs de douleur (anesthésiques, neutralisateur d'acidité, etc.) au médicament à injecter;
- diminution du coût de développement des dispositifs;
- possibilité d'ajustements dans la formulation du produit fluide.
- diverses formulations de produits fluides peuvent être logées dans un seul dispositif;
- réduction du nombre d'injections.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif d'injection automatique de produit fluide comportant un corps (1) fixé à un support (2) destiné à venir au contact d'une zone à injecter, ledit corps (1) contenant un ou plusieurs réservoirs (5) de produit fluide contenant chacun un piston d'injection (6), un ensemble d'aiguille (4) comportant une aiguille d'injection (40) destinée à pénétrer dans la zone à injecter, et un mécanisme d'injection (7, 8, 9, 10, 11), ledit mécanisme d'injection comportant une capsule d'actionneur (7) contenant un matériau expansible et des moyens de chauffage (9) pour chauffer et ainsi provoquer l'expansion dudit matériau expansible pour déplacer ledit piston (6) dans ledit réservoir (5) et ainsi injecter le produit fluide dans ladite zone à injecter à travers ladite aiguille d'injection (40), **caractérisé en ce que** ledit matériau expansible comprend des microsphères expansibles thermiquement.

2. Dispositif selon la revendication 1, dans lequel lesdites microsphères expansibles thermiquement comprennent des bulles thermoplastiques microscopiques avec un hydrocarbure encapsulé.

3. Dispositif selon la revendication 2, dans lequel lesdites bulles thermoplastiques microscopiques, après chauffage à 60-90°C, se dilatent et subissent un changement de phase en gaz, permettant l'expansion desdites microsphères expansibles thermiquement à 5-70 fois leur volume d'origine.

4. Dispositif selon l'une quelconque des revendications précédentes, comportant un ensemble d'amorçage (3) comportant un actionneur d'amorçage et une ou plusieurs aiguille(s) d'amorçage (31) destinée(s) à pénétrer dans le ou les réservoirs (5).

5. Dispositif selon l'une quelconque des revendications précédentes, dans lequel ledit support (2) comporte un autocollant pour se fixer sur la zone à injecter.

6. Dispositif selon l'une quelconque des revendications précédentes, dans lequel chaque réservoir (5) contient entre 1 et 10 ml de produit fluide, avantageusement environ 3 ml.

7. Dispositif selon l'une quelconque des revendications précédentes, dans lequel ledit corps (1) comporte plusieurs réservoirs (5), notamment trois.

8. Dispositif selon la revendication 7, dans lequel les produits fluides contenus dans lesdits réservoirs (5) sont distribués simultanément, en étant mélangé en amont de ladite aiguille d'injection (40).

9. Dispositif selon la revendication 7, dans lequel les produits fluides contenus dans lesdits réservoirs (5) sont distribués successivement.

10. Dispositif selon l'une quelconque des revendications précédentes, comportant des moyens d'alimentation de puissance, avantageusement une pile et/ou un accumulateur d'énergie électrique.

## Patentansprüche

1. Vorrichtung zum automatischen Einspritzen eines Fluidprodukts, die einen Körper (1) umfasst, der an einem Träger (2) befestigt ist, der dazu vorgesehen ist, mit einer Einspritzzone in Kontakt zu gelangen, wobei der Körper (1) einen oder mehrere Vorratsbehälter (5) für das Fluidprodukt, wovon jeder einen Einspritzkolben (6) enthält, eine Nadelanordnung (4), die eine Einspritznadel (40) aufweist, die dazu vorgesehen ist, in die Einspritzzone einzudringen, und einen Einspritzmechanismus (7, 8, 9, 10, 11) umfasst, wobei der Einspritzmechanismus eine Betätigungskapsel (7), die ein ausdehnbares Material enthält, und Heizmittel (9), um das ausdehnbare Material zu erwärmen und so dessen Ausdehnung hervorzurufen, umfasst, um den Kolben (6) in dem Vorratsbehälter (5) zu verlagern und somit das Fluidprodukt durch die Einspritznadel (40) in die Einspritzzone einzuspritzen, **dadurch gekennzeichnet, dass** das ausdehnbare Material wärmeausdehnbare Mikrokugeln enthält.

2. Vorrichtung nach Anspruch 1, wobei die wärmeausdehnbaren Mikrokugeln mikroskopische thermoplastische Bläschen, in die Kohlenwasserstoff eingekapselt ist, enthalten.

3. Vorrichtung nach Anspruch 2, wobei sich die mikroskopischen thermoplastischen Bläschen nach einer Erwärmung auf 60-90 °C ausdehnen und einer Änderung in die Gasphase unterworfen werden, was die Ausdehnung der wärmeausdehnbaren Mikrokugeln auf das 5-70-Fache ihres ursprünglichen Volumens ermöglicht.

4. Vorrichtung nach einem der vorhergehenden Ansprüche, die eine Auslöseanordnung (3) umfasst, die einen Auslöseaktor und eine oder mehrere Auslösenadeln (31), die dazu bestimmt sind, in den oder die Vorratsbehälter (5) einzudringen, umfasst.

5. Vorrichtung nach einem der vorhergehenden Ansprüche, wobei der Träger (2) ein selbstlebendes Element aufweist, um an der Einspritzzone befestigt zu werden.

6. Vorrichtung nach einem der vorhergehenden Ansprüche, wobei jeder Vorratsbehälter (5) zwischen 1 und 10 ml des Fluidprodukts, vorteilhaft etwa 3 ml, enthält.

7. Vorrichtung nach einem der vorhergehenden Ansprüche, wobei der Körper (1) mehrere Vorratsbehälter (5), insbesondere drei, enthält.

8. Vorrichtung nach Anspruch 7, wobei die in den Vorratsbehältern (5) enthaltenen Fluidprodukte gleichzeitig verteilt werden, indem sie stromaufseitig der Einspritznadel (40) gemischt werden.

9. Vorrichtung nach Anspruch 7, wobei die in den Vorratsbehältern (5) enthaltenen Fluidprodukte nacheinander verteilt werden.

10. Vorrichtung nach einem der vorhergehenden Ansprüche, die Leistungsversorgungsmittel, vorzugsweise eine Batteriezelle und/oder einen Akkumulator für elektrische Energie, enthält.

## Claims

1. An automatic fluid injection device comprising: a body (1) that is fastened to a support (2) for coming into contact with an injection zone, said body (1) containing one or more fluid reservoirs (5) each containing an injection piston (6), a needle assembly (4) including an injection needle (40) for penetrating into the injection zone, and an injection mechanism (7, 8, 9, 10, 11), said injection mechanism comprising an actuator cap (7) containing an expandable material and heater means (9) for heating said expandable material thus causing it to expand so as to move said piston (6) in said reservoir (5) and thus inject the fluid into said injection zone through said injection needle (40), the device being **characterized in that** said expandable material comprises thermally expandable microspheres.

2. A device according to claim 1, wherein said thermally expandable microspheres comprise hollow microscopic thermoplastic spheres encapsulating a hydrocarbon.

3. A device according to claim 2, wherein said hollow microscopic thermoplastic spheres, after heating to a temperature in the range 60°C to 90°C, expand and are subjected to a transformation into a gas phase, making it possible for said thermally expandable microspheres to expand in the range five times to seventy times their original volume.

4. A device according to any preceding claim, including a priming assembly (3) comprising a priming actuator and one or more priming needles (31) for penetrating into the reservoir(s) (5).

5. A device according to any preceding claim, wherein said support (2) includes a sticker for fastening onto the injection zone.

6. A device according to any preceding claim, wherein each reservoir (5) has a fluid content in the range 1 mL to 10 mL, advantageously about 3 mL.

7. A device according to any preceding claim, wherein said body (1) includes a plurality of reservoirs (5), in particular three reservoirs.

8. A device according to claim 7, wherein the fluids contained in said reservoirs (5) are dispensed simultaneously, being mixed together upstream of said injection needle (40).

9. A device according to claim 7, wherein the fluids contained in said reservoirs (5) are dispensed successively.

10. A device according to any preceding claim, including power supply means, advantageously an optionally rechargeable battery.
